Europäisches Patentamt

European Patent Office

Office européen des brevets

(11) Publication number: **0 485 471 B1**

(19)

(12) **EUROPEAN PATENT SPECIFICATION**

(45) Date of publication of patent specification: **27.04.94**     (51) Int. Cl.5: **C07K 15/26**, C07K 7/08, C07K 15/28, A61K 39/395

(21) Application number: **90912151.9**

(22) Date of filing: **08.08.90**

(86) International application number: **PCT/EP90/01293**

(87) International publication number: **WO 91/02005 (21.02.91 91/05)**

(54) NATURAL HUMAN ANTI-GAMMA INTERFERON ANTIBODIES DETECTED AND PURIFIED BY SYNTHETIC PEPTIDES.

(30) Priority: **11.08.89 IT 2151789**

(43) Date of publication of application: **20.05.92 Bulletin 92/21**

(45) Publication of the grant of the patent: **27.04.94 Bulletin 94/17**

(84) Designated Contracting States: **AT BE CH DE DK ES FR GB IT LI LU NL SE**

(56) References cited:
**EP-A- 0 103 898     EP-A- 0 168 745
EP-A- 0 304 291     WO-A-88/07869
US-A- 4 473 555     US-A- 4 599 306**

**J. Biol. Reg. Homeost. Agents 3, p. 8-12 (1989)**

(73) Proprietor: **TURANO, Adolfo
Viale Venezia, 82
I-25124 Brescia(IT)**

(72) Inventor: **TURANO, Adolfo
Viale Venezia, 82
I-25124 Brescia(IT)**

(74) Representative: **Bianchetti, Giuseppe
Studio Consulenza Brevettuale
Via Rossini, 8
I-20122 Milan (IT)**

Dialog Information Services, FILe 351, World Patent Index 81-90, Dialog accession no. 3853392, Takeda Chemical Ind. KK: "Novel polypeptide, protein complex and hybridoma having aminoacid sequence the same as part of interferon-gamma", JP 60107569, A, 850613, 8530 (Basic)

Dialog Information Services, File 351, World Patent Index 81-90, Dialog accession no. 3564867, Otsuka Pharm KK: "Peptide with partial amino acid sequence of gamma-interferon used to prepare specific antibody for gamma-interferon", JP 59122446, A, 840714, 8434 (Basic)

Dialog Information Services, File 154, Medline 83-90, Dialog accession no. 07082681, Lord SC et al: "Functional domains of human interferon gamma probed with antipeptid antibodies", Mol. Immunol Jul 1989, 26 (7) p. 637-40

Dialog Information Services, File 154, Medline 83-90, Dialog accession no. 05868687, Russell JK et al: "Epitope and functional specificity of monoclonal antibodies to mous interferon-gamma: the synthetic peptide approach", J. Immunol May 1 1986, 136 (9) p 3324-8

Dialog Information Services, File 154, Medline 83-90, Dialog accession no. 05713022, Leist T et al: "Antibodies to synthetic polypeptides corresponding to hydrophili regions of human interferon gamma" Mol Immunol Aug 1985, 22 (8) p 929-36

## Description

The invention relates to the use of human anti-gamma interferon antibodies in human therapy and to the detection and purification of said antibodies by means of synthetic peptides corresponding to regions of human gamma interferon.

## Background Art

Interferons (IFNs) form an heterogeneous family of proteins, defined according to their ability to prevent viral replication. At present three major classes of human IFN have been designed: human IFN-$\alpha$ and IFN-$\beta$ - which are 30% similar at the primary amino acid sequence level - and IFN-$\gamma$ which is similar to neither. IFN-$\gamma$ shares several characteristics and activities with IFN-$\alpha$ and IFN-$\beta$, but it also mediates various immune functions. More recently, IFN-$\gamma$ has been established to act as a potent immunomodulator (Table 1). Following antigenic stimulation, T-cells and NK cells release IFN-$\gamma$ which: influences the response of T-cells, B-cells and macrophages; enhances the proliferation of T-cells and the functional maturation of cytotoxic T-cells; inhibits the gereration of suppressor T-cells; enhances the immunoglobulin secretion of B-cells when added late during an in vitro immune response and switches on IgG2 a production. In addition to these regulatory effects on the adaptative immune response, IFN-$\gamma$ acts on the effector cells of nonadaptive defence and exerts proinflammatory activity. Macrophages, primed with IFN-$\gamma$ become able to kill bacteria, protozoa and tumor cells by oxygen-dependent and-independent mechanisms. In addition, IFN-$\gamma$ induces endothelial cells and monocytes to release chemiotactic factors, such as IL-1 and TNF and leads to enhancement of synthesis and surface expression of class I and class II antigens of the Major Histocompatibility Complex (MHC), Fc receptor and leukocyte adhesion proteins (e.g. integrins and integrin receptors). Induction of MHC antigens by IFN-$\gamma$ may occur in several cell types that otherwise express low or undetectable levels of these molecules. The enhancement of MHC class II expression enables macrophages, endothelial, epithelial and Langerhans cells to present antigens, but makes the same cells susceptible to the possible cytotoxic effects of class II restricted T-lymphocytes. The induction of Fc receptors increases the capacity of macrophages to phagocyte opsonized antigens. The enhancement of the surface density of integrin receptors promotes adhesion of monocytes to endothelial cells and to lymphocytes facilitating local inflammatory and immunological reactions.

While the general stimulatory activity of IFN-$\gamma$ in the progression of immunological and inflammatory responses indicates its potential therapeutical application as e.g. immunomodulant, the agents that are able to neutralize its activity may be beneficial when given to allotransplanted patients and to patients affected by autoimmune disorders, chronic inflammation, septic shocks or diabetes (Table 2) and any clinical state where an enhancement of IFN-$\gamma$ production is considered to be detrimental.

Antibodies are the most inherently specific natural immunosuppressive agents. Billiau (1988), Immunol. Today 9, 37, reported that murine anti-IFN-$\gamma$ antibodies experimentally inhibited the Shwartzman reaction. His experiments opened up interesting clinical applications for the treatment of Shwartzman-related or-like inflammatory reactions. Other data reported by Jacob et al. (1987), J. Exp. Med. 166, 798, showed that anti-IFN-$\gamma$ antibodies protect NZB mice against spontaneous development of autoimmune disease. Their experiments indicated that anti-IFN-$\gamma$ antibodies, policlonal or preferably monoclonal, may be candidated for trials in connective-tissue diseases, such as Systemic Lupus Erythematosus (SLE) and Rheumatoid Arthritis, Multiple Sclerosis, and possibly of all those diseases where activated cell-mediated immunity needs to be depressed. Antibody immunosuppressive therapy is used in certain instances in humans, for preventing Rh-related Erythoblastosis Fetalis. This treatment, however, is limited to diseases when the causative antigen is known, and a specific human antiserum is available.

As substances self-recognized by human immune system, IFNs should not elicit antibodies in man except in conjunction with autoimmune disorders or when their structure and antigenicity are modified. Antibodies to IFN-$\gamma$ were reported by Caruso et al. (1989) J. Biol. Reg. Homeost. Agents 3, 8, in patients infected with Human Immunodeficiency virus (HIV). More recently, Caruso et al. (1990) J. Immunol. 144, 685, reported the presence of natural antibodies to IFN-$\gamma$ in healthy individuals ranging from newborn babies to adults and, at higher levels, in patients suffering from different viral infections. Those antibodies specific to IFN-$\gamma$ were affinity-purified from sera taken from healthy individuals, and viral-infected patients or other different biological sources (See, "Modes for carrying out the invention"), by using a recombinant IFN-$\gamma$-coupled CNBr-activated Sepharose 4B column. The antibodies were found to be mainly of the IgG class, and mantained their ability ot bind recombinant IFN-$\gamma$. They did not neutralize antiviral activity of IFN-$\gamma$, while being capable of suppressing the IFN-$\gamma$ induction of class II MHC antigens and of Fc receptor sites for immunoglobulins. In addition, the natural human anti-IFN-$\gamma$ antibodies were found to interfere, in a mixed

3

lymphocyte culture (MLC), with the proliferation and cytotoxic generation of lymphocytes, by inhibiting endogenously produced IFN-γ. The availability of affinity purified human anti-IFN-γ antibodies, capable of neutralizing the immunomodulatory activity of IFN-γ, opens up interesting clinical applications, possibly for all those diseases where activated cell-mediated immunity needs to be regulated.

The ready availability of neutralizing human anti-IFN-γ antibodies may solve many of the problems associated with the administration of heterologous immunoglobulins, both polyclonal and/or monoclonal antibodies, which can induce anti-Ig antibodies.

The main objective of the present invention is to provide techniques to detect anti-IFN-γ antibodies and affinity-purify them, using synthetic peptides corresponding to regions of human IFN-γ.

## Disclosure of the Invention

The invention is based upon the discovery that synthetic peptides corresponding to regions of human IFN-γ can substitute the natural or recombinant IFN-γ proteins in detecting and purifying natural human anti-IFN-γ antibodies. Accordingly, the first aspect of the invention is the use of synthetic peptides in different immunological methods to detect human antibodies to IFN-γ. The above mentioned methods are based on the use of synthetic peptides capable to specifically bind anti-IFN-γ antibodies allowing their direct or indirect detection. The second aspect is a method to affinity-purify human anti-IFN-γ antibodies using synthetic peptides. The method is based on the binding of synthetic peptides to a support, or to other molecular carriers or on trapping the peptides into nitrocellulose sheets.

The third aspect of the invention is an immunotherapeutic treatment to control a disease associated with an activated cell-mediated immunity. The treatment consists in the administration of human antibodies directed against IFN-γ to the individual, sufficient to control the clinical aspects of the disease. The fourth aspect of the invention is a "unit dosage" for treatment of the above-described patients. The unit dosage form consists of human antibodies against IFN-γ combined with a pharmaceutically acceptable vehicle. The amount of human antibodies in the dosage form has to be sufficient to substantially lessen manifestation of the disease. Manifestation of the disease may be determined by clinical symptoms associated with the disease, and by the presence of auto antibodies associated with the disease while they are absent, or at lower titer, in healthy individuals.

The fifth aspect of the invention are immunological methods to detect the IFN-γ by the use of purified human anti-IFN-γ antibodies. The method comprises the use of human anti-IFN-γ antibodies to specifically bind and detect (in a direct or indirect manner) the IFN-γ molecule(s).

The last aspect of the invention is a method to affinity-purify the IFN-γ molecule(s) recognized by the human anti-IFN-γ antibodies. The method comprises binding of human anti-IFN-γ to a support, or to other molecular carriers or trapping them into nitrocellulose sheets.

## Brief description of the Drawing

Figure 1 represents a graph showing the inhibition of the expression of Fc-receptor sites and HLA-DR antigens on U937 cells stimulated with recombinant IFN-γ by human anti-IFN-γ antibodies.

Figure 2 presents a graph showing the effect of human anti-IFN-γ antibodies, added at different times, on MLC proliferation.

Figure 3 presents a graph showing the effect of human anti-IFN-γ antibodies on the development of cytotoxicity in MLC.

Figure 4 presents the chromatography of human anti-IFN-γ antibodies on a IFN-γ-relating peptide-sulfolinked agarose column.

Figure 5 presents a Western blot analysis of the anti-IFN-γ antibodies affinity purified by an IFN-γ-relating peptide based column showing their specific reactivity with IFN-γ.

Figure 6 presents a graph showing the effect of human anti-IFN-γ antibodies purified by a peptide-based affinity column of MLC proliferation.

Figure 7 presents a graph showing the correlation obtained by an IFN-γ-relating peptide based ELISA and a recombinant IFN-γ based RIA in detecting and quantify human anti-IFN-γ antibodies in human serum.

## Modes for Carrying Out the Invention

The practice of the present invention will employ, unless otherwise indicated, conventional techniques of molecular biology, microbiology, recombinant DNA, chemistry, biochemistry, biotechnology and immunology, which are within the skill of the art. Such techniques are explained fully in the literature (See Literature, Appendix 1).

According to the invention, it is very useful and of relevance for scientists and clinicians, to detect and quantify natural antibodies to IFN-γ in specimens by any of the known assays.

For example, assays to detect human anti-IFN-γ antibodies may be useful:

a) in clinic, for a rapid diagnosis of viral diseases, and to distinguish between a viral and bacterial infection; to monitor a viral infection and, eventually, a specific antiviral therapy; to study anti-IFN-γ antibodies in patients with cancer, viral infections, and/or immunological disorders; to monitor organ transplantation, for a rapid diagnosis of viral infections in transplanted patients, in order to prevent an eventual allotransplant rejection; to monitor antiviral as well as immunomodulating therapies;

b) in industry, for the quality control of immunoglobulin preparations obtained by chromatography or other preparative methodologies;

c) in blood bank, to detect viral infections in apparently healthy blood donors.

**Immunological assays**

Assays for detection of antibodies to IFN-γ may be developed using natural and recombinant IFN-γ, or IFN-γ-relating peptides of various lenghts, which are representative of the different epitopes of the original molecule. These assays may be based on any of the classical RIA and ELISA techniques.

Microtiter plates, strips, wells or other solid phase supports may be coated with the antigen with any suitable technique known in the art, like overnight incubation at various temperatures in appropriate buffers. The antigen used for coating may be a single protein/peptide or a mixture of different antigens depending upon the antibodies which have to be detected. Antibodies may be detected in several specimens like plasma, serum, cerebro spinal fluid, urine, saliva, tissue culture fluids etc.

After incubation of the specimens with the antigen, the bound antibody can be detected by addition of an enzyme conjugated antiserum or monoclonal antibody. All the traditional enzyme tracers can be employed in this assay (horse radish peroxidase, alkaline phosphatase, etc.) and the sensibility of the assay may be improved by use of any biotin-avidin or streptavidin system for detection of the analyte. The final detection can be achived by addition of a substrate solution which is variable depending upon the enzyme used.

Examples of idoneous substrate are o-phenylendiamine, tetramethylbenzidine, paranitrophenylphosphate and others. Results can be read by eye or by a spectrophotometer and the assay can be both qualitative or quantitative. Quantification can be obtained by end point dilution, standard curve system or by any other suitable quantification technique.

Any principle of assay can be used for the development of an ELISA assay for detection of antibodies and particularly they could be homogeneous assays or eterogeneous assays like classical or modified competition assays, titration assays, direct and indirect sandwich-assays, Ig capture assays and the like.

The same principles used for detection of antibodies by ELISA may be used by RIA or time resolved fluoroimmunoassay or other assays based on the use of different tracers.

At the state of the art, antisera and monoclonal antibodies obtained using natural and recombinant IFN-γ can be used to develop assays for detection of IFN-γ in tissue culture fluids and in biological specimens. Purified antibodies to IFN-γ obtained by affinity chromatography or other purification procedures, may be used as well as antisera and monoclonal antibodies and/or together with these reagents, for development of antigen detection assays. All the conventional thchniques may be applied to develop antigen detection assays which may be, for example, capture or competition immunoassays (RIA, ELISA, TR-FIA etc.). To perform competition immunoassays natural recombinant IFN-γ, or IFN-γ-relating peptides can be used. They may be conjugated to various tracer molecules. For example, they may be conjugated to enzymes suitable for ELISA techniques, radioactive materials for RIAs, fluorescent molecules for TRFIA and other immunofluorescent techniques, and others tracers. Detection of antigen may also be obtained by immunofluorescence, flow cytometry or other techniques known in the art.

**Peptides**

The general methodologies for obtaining peptides are well known. Peptides of IFN-γ may be obtained using different methods:

a) Enzyme digestion and chemical cleavage of natural or recombinant proteins. See, e.g., Arakawa et al. (1986), J. Biol. Chem. 261, 8534; and Seeling et al. (1988), Biochem. 27, 1981.

b) Enzyme catalized synthesis in vitro. See, e.g., Mitin and Zapevalova (1990), Int. J. Peptide Protein Res. 35, 352.

5

c) Enzime modification of analogues.

d) Synthesis by recombinant techniques. See, e.g., Charbit et al. (1987), J. Immunol. 139, 1658.

e) Site-specific and regionally directed mutagenesis of protein-encoding sequences. See, e.g., Kunkel (1985), Proc. Natl. Acad. Sci. U.S.A., 82, 488.

f) Solution peptide synthesis. See, e.g., Bodansky (1984) in Principles of Peptide synthesis, Springer-Verlag, Heidelberg; Bodansky (1984), The practice of peptide synthesis, Springer-Verlag, Heidelberg.

g) Solid-phase peptide synthesis. See, e.g., Sheppard (1989), Solid Phase Peptide Synthesis, IRL Press, Oxford.

h) Segment condensation. See, e.g., Pettit (1976), in Synthetic Peptides, Vol. 4, p22, Elsevier, Amsterdam.

i) And, in general, all the appropriate combinations of the above mentioned methods.

The 146-amino acid sequence of mature human IFN-γ, deduced from the nucleotide sequence of a cloned cDNA is well known. See, e.g., Gray et al. (1982) Nature, 295, 503, Grey and Goeddel (1982), Nature 298, 859.

IFN-γ-relating peptides of different lenghts may be synthesized. For example, we have obtained IFN-γ-relating peptides, spanning 14 residues which, at 7 amino acids intervals, which cover the native IFN-γ primary sequence from amino acid 121 (Ala) to amino acid 140 (Gln) (Table 3). This list is not meant to be exhaustive, and shorter or longer peptides, may be obtained, combined, and used to detect or purify natural human anti-IFN-γ antibodies.

Provided that at least 5 aminoacids are present whereas the upper limit is not critical but practical reasons limit the maximum number of aminoacids to about 20. IFN-γ -relating peptides may have C-terminal functions as free acid, alcohol, amide, ester, hydrazide, etc; and N-terminal function modified. A number of techniques to modify the N-terminal function are known in the art, the most common are being acylation, alkylation, etc.

Some amino acid residues forming IFN-γ-relating peptides may be repeated, deleted or substituted in a conservative or nonconservative manner. For example, amino acids from the native structure of IFN-γ may be substituted by other uncorrelated amino acids that mimic the tertiary structure of the epitope, forming a mimotope; amino acid residues may also be replaced by pseudoisosteric aminoacids. Morover Methionine can be replaced by N-Leucine or N-ethyl-Norleucine. Relevant sequences may be also inserted into peptide skeleton to increase its affinity to antibodies and its stability.

Peptides can also be conveniently glycosilated by different sugars or modified sugar molecules. A number of techniques for obtaining glycosilation of peptides are known in the art. Peptides of IFN-γ may be also modified in a way that alter their backbone conformation. For example, conformational constraints may be obtained inserting in the peptide chain D-amino acids, alpha methyl amino acids, Proline and other amino acids, even modified. See, e.g., Marshall and Bassbard (1972), Circ. Res., Suppl., Suppl. II to 30, 41, 143; Manavalan and Momany (1980), Biopolymers 19, 1943; Madison and Kopple (1980), J. Am. Chem. Soc. 102, 4855.

Other possibilities of backbone modification may include the C7 turn mimics of Huffman & Callahan, the β-turn mimics of Freidingen, Kahn, Kemp and others. See, e.g., Huffman et al. (1988). Peptides: Chemistry and Biology. Proc. 10th. American Peptide Symp. (Marshall, G.R. ed.) pp 105-108, ESCOM, Leiden; Freidingen, (1981) Peptides: Synthesis, Structure and Function, Proc 774 American Peptide Symposium (Rich, D.H. & Gross, E., eds), pp. 673-783, Pierce Chemical Co., Rockford, IL; Kahn (1988) Peptides, Chemistry and Biology, Proc 10th American Peptide Symp. (Marshall, G.R., ed.) pp. 109-111, ESCOM, Leiden; Kemp & Sun (1982) Tetrahedron Lett. 23, 3759.

Peptide bonds may be modified so as to induce conformational restriction or to increase stability to enzymatic attack.

Peptide bonds modification can be either $-C^{\alpha}$ H< and -CO-NH- without altering the N-C-C backbone atomic sequence, for example $C^{\alpha,\alpha}$ - disubstituted peptides, -β-dehydropeptides, thiated peptides, N-alkylated peptides, N-hydroxylated peptides, nitrono peptides etc. Further alterations may be obtained by N-C-C backbone modification for example to form compounds containing α-hydroxy- or ω-amino acids; $[\psi CH_2 O]$; $[\psi CH (OH) CH_2]$; $[\psi CH_2 -NH]$; $[\psi CH = CH]$. These peptide surrogates or others can be used. See e.g., Spatola (1983) Chemistry and Biochemistry of Amino Acids Peptides and proteins (Weinstein, B. ed.) Vol 7, pp 267-357, Dekker, NY, McQuade et al (1990), Science, 247, 454, Miller et al, (1989). For nomenclature see, e.g., IUPAC-IUB Commission in Biochemical Nomenclature (1984) European J. Biochem., 138, 9. On the other hand IFN-γ-relating peptides can be conformationally restricted by either short-, medium- and long-range cyclization to form homodetic cyclic peptides, heterodetic cyclic peptides, bicyclic systems for instance through N↔C', C ↔C , C ↔C', N↔C , C'↔C', N↔N, or spiro-system formation, or even by other techniques known in the art. See, e.g., Toniolo, 1990, Int. J. Peptide Protein Res. 35, 287.

Nucleotides, nucleosides deossinucleotides oligo- and polinucleotides, lipides, glycolipids, terpens and their analogs can be introduced into the IFN-γ-relating peptide sequences.

Lipids, glycolipids, terpens, and appropriate derivates of these compounds may be bound to the IFN-γ-relating peptides. INF-γ-relating peptides may also be formed by various combinations of modified or unmodified peptides, obtainable by different techniques known in the art.

## Carriers

Peptides may be linked, for different purposes (for example, to facilitate their binding to a support), to a suitable carrier to form a conjugate. Any carrier may be used, such as the various serum albumins, tetanus toxoids, or keyhole limpet hemocyanin (KLH) as in the state of the art.

Peptide-protein conjugates can be obtained basically using simmetrical or asymmetrical bifunctional reagents. They may be incorporated into the final conjugate or may activate certain reactive sites on one molecule for the subsequent linkage with the other one.

A number of techniques for obtaining such linkage are known in the art, including glutaraldehyde, bis imido esters, carbodiimides, imido esters, toluene diisocyanate, p-nitrobenzoyl chloride, hystamine dihydrochloride, MBS (Maleimidobenzoyl-N-hydroxysuccimimide) and many others. Point of attachment of carrier molecules on a peptide can be different, depending on the peptide structure and the steric requirements. If the peptide lacks a sulphydryl, this can be provided by addition of a cysteine residue. These reagents create a disulfide linkage between themselves and peptide cysteine residues on one side, and an amide linkage through the ε-amino on a Lysin or other free amino group on the other. A variety of such disulfide/amide-forming agents are known. See, e.g., Immun. Rev. (1982) 62, 185. Other bifunctional coupling agents form a thioether rather than a disulfide linkage.

Binding of INF-γ-relating peptides to the carrier molecule may be achived by non covalent bonds. For example, a peptide may be linked to a carrier protein through a hydrophobic domain in a hydrophobic poket.

## Purification

Human natural antibodies directed against IFN-γ, can be purified from different specimens, including plasma, serum, urine, saliva, or already purified human immunoglobulin preparations. Human immunoglobulins may include different classes (IgM, IgG, IgA, IgD or IgE). They may be in the native structure or denaturated under a variety of experimental conditions knows in the art., or may include antigen binding fragments (F(Ab')$_2$, Fab, Fab', FV) of immunoglobulins. The antigen binding fragments of immunoglobulins may be obtained chemically, enzymatically, or by recombinant DNA techniques, e.g. miniantibodies.

Human natural antibodies to IFN-γ can be purified by any of the known affinity techniques, using IFN-γ or IFN-γ-relating peptides as specific binding molecules linked to a support.

One of the many techniques known in the art, is the affinity chromatography.

IFN-γ-relating peptides may be linked to many of the known matrices including Agarose, silica gel, polyacrylamide, and the like.

Binding of peptides to the matrix may be achieved cross-linking the functional groups on the matrix and on the peptide by interposition of a spacer arm. Spacer arms most often are linear aliphatic C6 - C8 chain with functional groups able to form a bridge between matrix and peptide. Both hydrophilic compounds (i.e., alcohols) and hydrophobic compounds (i.e. spacer arms including a benzene ring or others groups) can be used. Carrier molecules can be used instead of spacer arms.

In order to avoid denaturation of the peptides following their attacheament to a matrix and to facilitate this process, it may be useful to preactivate the matrix so that subsequent binding of the ligand may be achieved under mild conditions. Activation is a chemical reaction between the matrix and the activating compound, thus resulting in the formation, at the surface of the matrix itself, of reactive groups (usually electrophilic) which readily combine with groups of the ligand (usually nucleophilic, e.g. amino groups). Reactive groups such as imidocarbonate, oxirane (epoxy-activation), trichlorotriazine, O-imidazolylcarbonyl, and many others are known in the art. By reacting with an N-hydroxysuccinamide ester of bromoacetic acid, a spacer arm with a terminal amino group may be activated to give rice to a highly reactive alkylating agent.

Ready to use matrices with activated spacer arms may be used, such as, for example, SulphoLink coupling gel, in which the active site is a iodoacetil group that readely react whith a free sulphydryl group.

IFN-$\gamma$-relating peptides may be also bound to a matrix by non covalent bonds using, for example, a triazine dye resin. Moreover, peptides of IFN-$\gamma$ may also be bound to a matrix by reversible covalent bonds.

On the other hand, purified human antibodies to IFN-$\gamma$ may also be linked to a support to affinity-purify the recognized form(s) of IFN-$\gamma$.

Affinity linkage between antibodies and IFN-$\gamma$ to the affinity matrix can be broken either directly, by creating conditions which are unfavourable for biospecific interactions or by means of competitive affinity elution.

### Therapy

Murine anti-IFN-$\gamma$ antibodies were shown to prevent the rejection of allogeneic tumor cells, to inhibit the Shwartzman-related or-like reaction, and to protect NZB mice against spontaneous development of autoimmune diseases. See, e.g., Landolfo et al. (1985), Science 229, 176, Billiau (1988), Immunol. Today 9, 37, Jacob et al. (1987), J. Exp. Med. 166, 798.

Purified human antibodies to IFN-$\gamma$ may be used in clinic as specific antagonists of IFN-$\gamma$ to selective immunosuppress the physiological response(s) induced by IFN-$\gamma$, and more specifically, those responses which are involved in the up-regulation of the immune or autoimmune process. Being proteins, the antibodies will be administered parentally, preferably intravenously. Since they may react with white blood cells, they will preferably be administered slowly, either from a conventional IV administration set or from a subcutaneous depot. The dose for individuals, and for different diseases, is determined by measuring the effect of the anti-IFN-$\gamma$ antibody on the lessening of those parameters which are indicative of the disease being treated. Based on the experience of Jacob et al. (1988), J. Exp. Med. 166, 798, and considering the natural clearance of antibodies, the dose of human anti-IFN-$\gamma$ antibodies may have to be repeated periodically depending upon the particular disease. When used as prophylaxis, it may be possible to administer short courses of human anti-IFN-$\gamma$ antibodies bimonthly, semiannually or annually. In treating an existing disease it is expected a most frequent antibody administration, also using infusion devices. For autoimmune diseases that are known to be triggered or aggravated by particular environmental factors which increase the level of IFN-$\gamma$, the dosage regimen will be scheduled accordingly.

When administered parentally, the human anti-IFN-antibodies will be formulated in a unit dosage injectable form (solution, suspension, emulsion) in association with a nontoxic and nontherapeutic acceptable parental vehicle. Nonacqueous vehicles may also be used. The vehicle may contain substances that enhance isotonicity and chemical stability. The antibody is preferably formulated in purified form, substantially free of aggregates and other proteins, at various concentrations ranging approximately from 0.5 mg/ml to 20 mg/ml.

### Examples

The following examples further illustrate the invention. These examples are not intended to limit the scope of the invention. In light of the present disclosure, numerous embodiments within the scope of the claims will be apparent to those of ordinary skill in the art.

I. Inhibition of natural and recombinant IFN-$\gamma$-induced Fc receptor and HLA-DR antigens by affinity-purified human anti-IFN-$\gamma$ antibodies.

U937 cells, when incubated in a medium containing natural or recombinant IFN-$\gamma$, increase both Fc receptor and HLA-DR antigens on their surface. The maximal effect is usually reached at 24h, using IFN-$\gamma$ at a concentration of 200 U/ml.

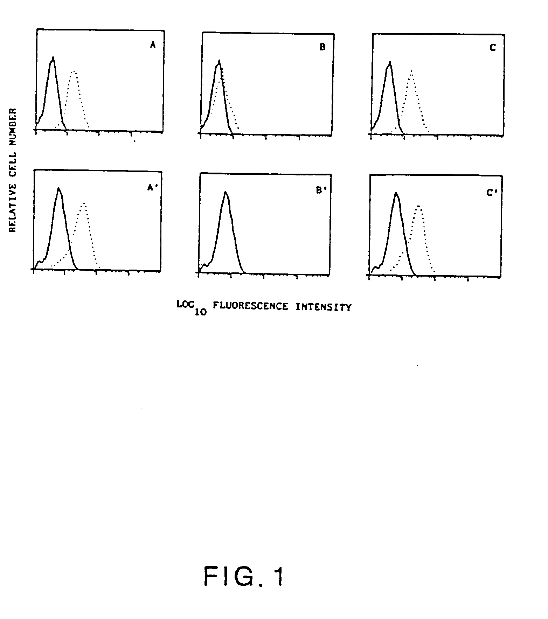

As shown in figure 1, when affinity-purified human anti-IFN-$\gamma$ antibodies were present in U937 cell cultures stimulated with IFN-$\gamma$, they dramatically inhibited the expected increase in Fc receptor and HLA-DR antigens. The inhibition was found to be dose-dependent (Table 4) maximally effective with human anti-IFN-$\gamma$ antibodies at a concentration of 4.8 $\mu$g/ml. At the same time no inhibitory effect was observed when unrelated purified human immunoglobulins were added, as a control, to IFN-$\gamma$ treated U937 cell cultures.

Expression of Fc receptor sites (A, B, C), and HLA-DR antigens (A', B', C') on U937 was evaluated by flow cytometric analysis.

Continuous line: cultures set up in the absence of IFN-$\gamma$. Dotted line: cultures set up in the presence of IFN-$\gamma$ (A, A'), in the presence of IFN-$\gamma$ and human anti-IFN-$\gamma$ antibodies (B, B'), and in the presence of IFN-$\gamma$ and human unrelated immunoglobulins (C, C').

I.A.1. Effect of human anti-IFN-$\gamma$ antibodies on the lymphocyte proliferation induced by irradiated allogeneic peripheral blood leukocytes (PBL).

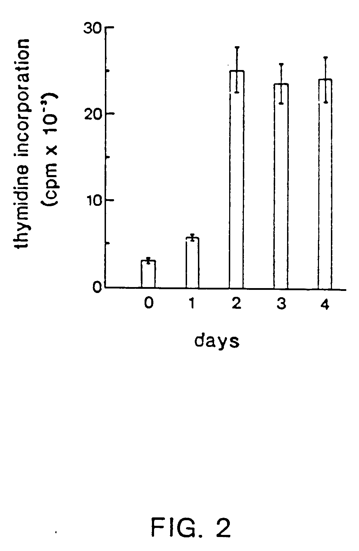

On day 7, mixed lymphocyte cultures result in marked lymphocyte proliferation. The addition of human anti-IFN-γ antibodies at the beginning of the culture resulted in a markedly reduced uptake of [³H]-thymidine. The inhibiting ability of human anti-IFN-γ antibodies was gradually lost with decreasing concentrations of antibodies (Table 5). Figure 2 shows that lymphocyte proliferation was inhibited when human antibodies specific to IFN-γ were added on days 0 and 1. When the same antibodies were added later (days 2, 3, and 4), no inhibition in proliferative response was observed. Purified human anti-IFN-γ antibodies were added at the concentration of 4.8 μg/well at the initiation of mixed lymphocyte culture or on day 1, 2, 3, 4. [³H)thymidine pulse for 18h on day 6.

I.A.2. Effect of human anti-IFN-γ antibodies on allogeneic induced lymphocyte cytotoxicity.

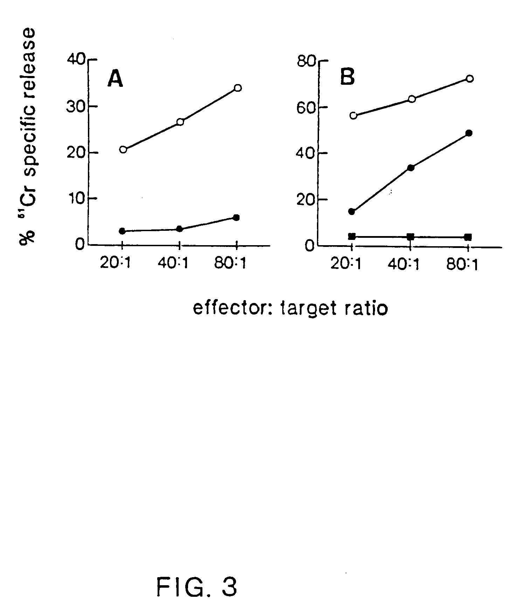

Figure 3 shows a representative experiment on the cytotoxic response of mixed lymphocyte cultures, evaluated on PHA-stimulated PBL or on K562 cells. The cytotoxicity of effector cells, recovered from cultures set up in the presence of human anti-IFN-γ antibodies, was strongly reduced against PHA-stimulated PBL. On the other hand, the human anti-IFN-γ antibodies had only a moderate influence on the development of cytotoxic lymphocytes to K562 cells. In other experiments, the cytotoxic activity to K562 cells was reduced to only 10-20% as compared to control cultures.

PBL were activated in vitro with irradiated allogeneic PBL. Purified human antibodies to IFN-γ (●) or unrelated purified human immunoglobulins (◎) were added, at the concentration of 4.8 μg/well, at the initiation of mixed lymphocyte culture. (■) PBL kept with medium only. Target cells were PHA stimulated lymphocytes (A) and K562 (B).

II. Chromatography of human anti-IFN-γ on an agarose matrix cross-linked through a spacer arm to peptide n° 22 (See Table 3).

Two mg of peptide n° 22, provided with an additional L-cysteine at its N terminal, was linked to 2 ml of Sulfolink coupling gel (PIERCE) following a standard protocol recommended by PIERCE. The affinity column was connected to an FPLC apparatus (PHARMACIA) and equilibrated with phosphate buffered saline (PBS). Purified human antibodies (50 mg) were applied to the column at a flow rate of 0.1 ml/min., and the bound antibodies were eluted at the same flow rate with 0.1 M glycine, pH 3.0.

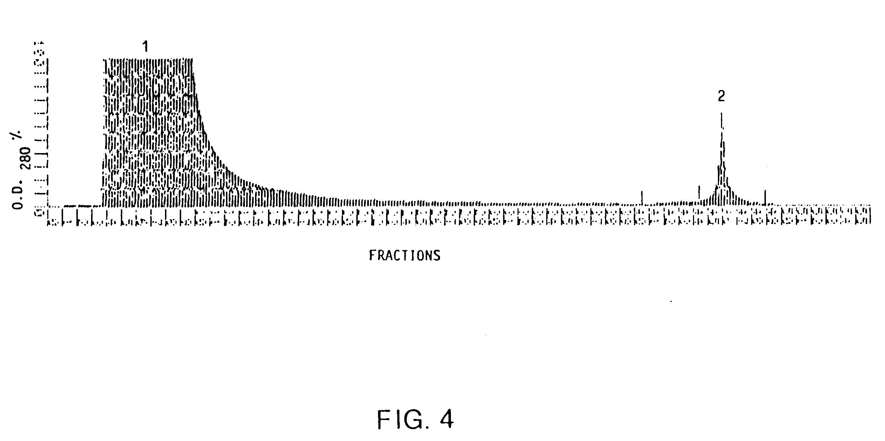

Figure 4 shows a typical pattern of elution of antibodies bound to the affinity matrix (See, peak 2).

III. Western blot analysis of the affinity-purified human anti-IFN-γ antibodies.

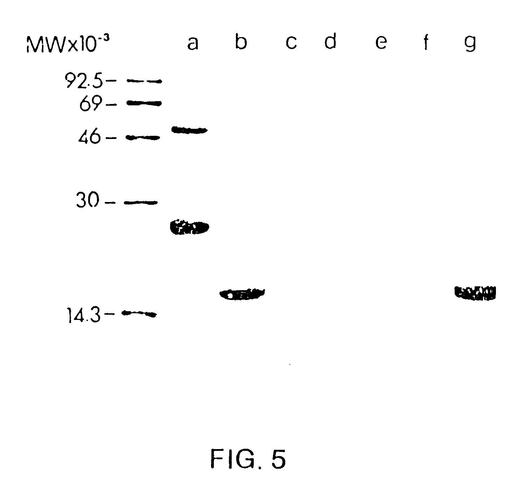

Antibodies specifically reacting to IFN-γ, and purified on a IFN-γ-relating peptide based affinity column, were confirmed to be immunoglobulins and to react to recombinant IFN-γ by Western blot analysis. Figure 5 shows that by using ¹²⁵I-conjugated goat anti-human Ig as a tracer, the anti-IFN-γ antibodies, in a denatured form, were recognized as two reactive bands of 25,000 and 50,000 molecular weight, being the light and heavy chain of immunoglobulins respectively (a). At the same time, these antibodies were capable of reacting with recombinant IFN-γ proteins of 16,000 and 32,000 molecular weight (Hoffmann-La Roche) (b). The specificity of the recombinant IFN-γ reactivity of purified human antibodies was confirmed by comparing it with the reactivity of a commercially available anti-IFN-γ monoclonal antibody (Boehringer) (g).

Human anti-IFN-γ antibodies did not react with natural IFN-α (gift of Dr. Kari Kantell) (b), natural IFN-β - (Serono) (c), recombinant IFN-α (Hoffmann-La Roche) (e), and recombinant interleukin-2 (Boehringer) (f).

IV. ELISA for detection of anti-IFN-γ antibodies in human serum.

Wells of polystyrene microtitration plates were coated with IFN-γ-relating peptides or recombinant IFN-γ. In order to quantify the amount of specific anti-IFN-γ antibodies in the specimens, a negative control and a series of positive controls were included in each assay. The positive controls were given a value of 1, 25, 30, 50, 60 and 100 arbitrary antibody units (AU). A standard curve was plotted for each test run referring to the adsorbance of these controls and each test specimen was given a value in AU based on such standard curve.

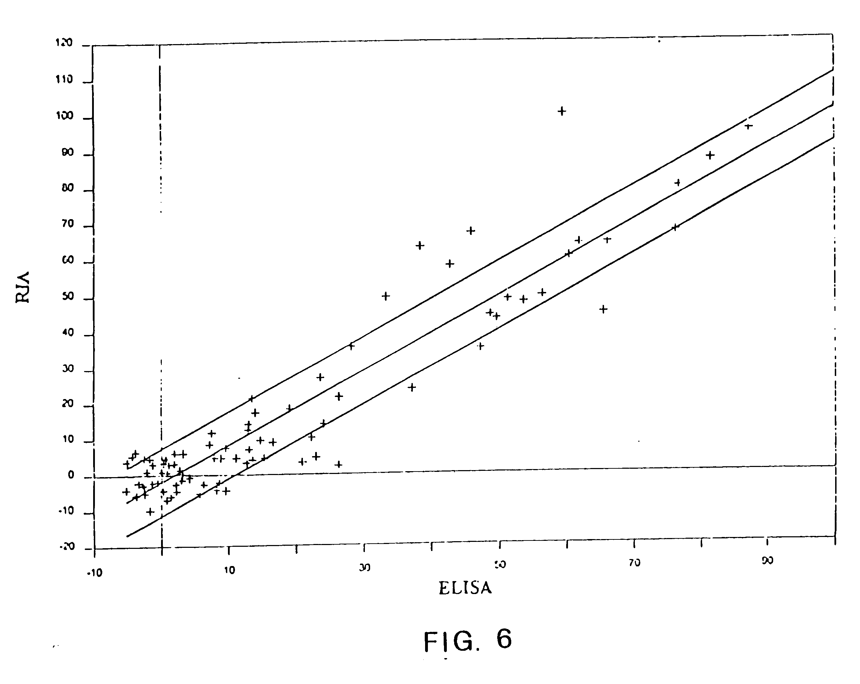

A panel of 88 serum specimens was used to study the correlation of the results obtained from an ELISA based on an IFN-γ-relating peptide (peptide n° 21 in this example; See, Table 3) and a RIA based on recombinant IFN- as antigen on the solid phase. As shown in figure 6, the correlation between ELISA and RIA was very high, with a correlation coefficient of 0,94, and 80,5% of the results folling within ± 1SD from the theoretical values indicated by the linear regression line.

## Utility

The various embodiments of the invention are useful for the detection, quantitation, purification of human anti-IFN-γ antibodies, and for treatment of individuals susceptible to autoimmune diseases or, more in general, of individuals suffering from all those diseases where activated cell-mediated immunity needs to be depressed.

**APPENDIX 1**

<u>Literature</u>

Maniatis, Fritsch and Sambrook, MOLECULAR CLONING: A LABORATORY MANUAL (1982); DNA CLONING, Volumes I e II (D.N. Glover ed. 1985); OLIGONUCLEOTIDE SYNTHESIS (M.J. Gait ed. 1984); NUCLEIC ACID HYBRIDIZATION (B.D. Hames and S.J. Higgins; eds. 1984); ANIMAL CELL CULTURE (R.K. Freshney, ed. 1986); IMMOBILIZED CELLS AND ENZYMES (IRL Press, 1986); B. Perbal, A PRACTICAL GUIDE TO MOLECULAR CLONING (1984); The series, METHODS IN ENZYMOLOGY (S. Clowick and N. Kaplan, eds., Academic Press, Inc.), and HANDBOOK OF EXPERIMENTAL IMMUNOLOGY, Volumes I-IV (D.M. Weir and C.C. Blackwell, eds., 1986, Blackwell Scientific Publications); M. Bodanszky, PRINCIPLES OF PEPTIDE SYNTHESIS (1984); K. March, ADVANCED ORGANIC CHEMISTRY (1988); THE PEPTIDES, ANALYSIS, SYNTHESIS, BIOLOGY (E. Gross and J. Meienhofer, eds., 1980, Acxademic Press, Inc.); P. Tijssen, PRACTICE AND THERAPY OF ENZYME IMMUNOASSAYS (R.H. Burdon and P.H. Van Knippenberg, eds., 1988, Elsevier); L.A. Osterman, METHODS OF PROTEIN AND NUCLEIC ACID RESEARCH, Volumes 1-3 (Springer-Verlag); PROTEIN ENGINEERING (D.L. Oxender and C.F. Fox, eds., 1988, Alan R. Liss, Inc.).

Table 1

| The IFN-$\gamma$ multiple activities. | |
|---|---|
| T-lymphocytes | Promotes T lymphocytes proliferation. Induces maturation of cytotoxic T lymphocytes. Inhibits maturation of suppressor T lymphocytes |
| B-lymphocytes | Promotes Ig synthesis and switch to IgG2a Inhibits IgE synthesis |
| Macrophages | Induces or increases the expression of MHC class II, Fc receptor, Integrin receptors Mac-1, LFA-1, CR3; induces synthesis of IL-1, TNF, chemotactic factor. Increases release of proteolytic enzymes. Activates the oxidative burst and killing of microorganisms and tumor cells. |
| Endothelial cells | Induces the expression of MHC class II and ICAM-1 and release of chemotactic factors. |
| Epithelial cells | Induces the expression of MHC class II and ICAM-1. |
| PMN | Enhances the release of proteolytic enzymes and oxidative radicals. |

Table 2

| Disease known to benefit of a therapy with IFN-$\gamma$ antagonists. | |
|---|---|
| Type I Diabetes | Delayed by immunosuppressive agents. Exacerbated by IFN-$\gamma$. |
| Multiple Sclerosis | Exacerbated by IFN-$\gamma$. |
| Lupus-Erythematosus | Delayed by anti-IFN-$\gamma$ MAbs. Development of nephritis exacerbated by IFN-$\gamma$. |
| Adjuvant Arthritis | Exacerbated by IFN-$\gamma$ (early phase of the disease) and delayed by anti-IFN-$\gamma$ MAbs. |
| Shwartzman Reaction | Lethal effects of endotoxin, thrombosis and hemorrhagia prevented by anti-IFN-$\gamma$ MAbs. |
| Delayed hypersensitivity | Local recruitment of T cell inhibited by anti-IFN-$\gamma$ MAbs. |
| Allotransplant Rejection | Rejection of tumor skin and heart allografts delayed or blocked by anti-IFN-$\gamma$ MAbs. |

Table 3. Sequences of peptides of the invention

21 - Ala-Glu-Leu-Ser-Pro-Ala-Ala-Lys-Thr-Gly-Lys-Arg-Lys-Arg.   (aa. 121-134).

22 - Ala-Lys-Thr-Gly-Lys-Arg-Lys-Arg-Ser-Gln-Met-Leu-Phe-Gln.   (aa. 127-140).

Table 4

| Effect of natural human antibodies to IFN-$\gamma$, added at different concentrations on the expression of Fc receptor and HLA-DR antigens on U937 cells stimulated with rIFN-$\gamma$ ( a). | | | | |
|---|---|---|---|---|
| Addition to culture | % of Fc receptors-expressing U937 cells | Inhibition (%) | % of HLA-DR expressing U937 cells | Inhibition (%) |
| Antibodies to IFN-($\mu$g/ml) | | | | |
| 0 | 83 | - | 74.1 | - |
| 0.60 | 84.2 | 0 | 71.4 | 3.7 |
| 1.20 | 60 | 27.8 | 47.4 | 36.1 |
| 2.40 | 30.4 | 63.4 | 13.6 | 81.7 |
| 4.80 | 8.4 | 89.9 | 1.2 | 98.4 |
| Unrelated antibodies ($\mu$g/ml) | | | | |
| 4.80 | 83.6 | 0 | 73.5 | 0.7 |

a. U937 cell cultures were stimulated for 24 hr with 200 U of rIFN-$\gamma$ and at the same time, they were treated or not with different concentrations of human anti-IFN-$\gamma$ antibodies, or with purified human unrelated Ig.

Table 5

| Effect of human anti-IFN-γ antibodies added at different concentrations on allogeneic induced lymphocytic proliferation ( a). | | |
|---|---|---|
| **Addition to culture** | **[³H] thymidine incorporation (cpm ± SD)** | **Inhibition (%)** |
| **Exp. n. 1** | | |
| Antibodies to IFN-γ (μg/well) | | |
| 0<br>4.80<br>2.40<br>1.20<br>0.60 | 7202±422<br>216± 64<br>4837±312<br>7485±453<br>7334±375 | -<br>97<br>32.9<br>0<br>0 |
| Unrelated antibodies (μg/well) | | |
| 4.80 | 7180±483 | 0.3 |
| **Exp. n. 2** | | |
| Antibodies to IFN-γ (μg/well) | | |
| 0<br>4.80<br>2.40<br>1.20<br>0.60 | 6182±283<br>374± 77<br>5196±188<br>6327±312<br>5986±507 | -<br>94<br>16<br>0<br>3.2 |
| Unrelated antibodies (μg/well) | | |
| 4.80 | 6303±358 | 0 |

a. Lymphocytes admixed with irradiated allogeneic PBL, at a responder: stimulator ratio of 1:1, were incubated with medium alone or with medium containing different ratios of human antibodies to IFN-γ, or of unrelated human antibodies. Proliferative response was measured on day 7 after 18 hr pulse with [³H) thymidine.

**Claims**

1. Peptides selected in the group of
Ala-Glu-Leu-Ser-Pro-Ala-Ala-Lys-Thr-Gly-Lys-Arg-Lys-Arg. (aa. 121-134).
Ala-Lys-Thr-Gly-Lys-Arg-Lys-Arg-Ser-Gln-Met-Leu-Phe-Gln. (aa. 127-140).

2. Use of the peptides of claim 1, for the determination of anti-IFN-γ antibodies in the serum.

3. Use according to claim 2 wherein the determination is carried out by RIA, ELISA, fluoroimmunoassay methods.

4. Use of the peptides of claim 1 for the affinity-purification of anti-IFN-γ antibodies.

5. Use of human anti-IFN-γ antibodies for the preparation of a medicament for the treatment of conditions which may benefit of a selective immuno suppression of the pathological responses induced by IFN-γ.

6. Use according to claim 5 wherein said conditions are type I Diabetes, Multiple sclerosis, Lupus erythematosus, Adjuvant arthritis, Schwartzamnn reaction, delayed hypersensitivity, allotransplant rejection.

7. A pharmaceutical composition containing as active principle human anti-IFN-γ antibodies.

8. Pharmaceutical composition according to claim 7 in unit dose form.

9. Use of human anti-IFN-γ antibodies for the purification of natural or recombinant IFN-γ.

10. Use of human anti-IFN-γ antibodies for the immuno-analysis of IFN-γ.

**Patentansprüche**

1. Peptide ausgewählt aus der Gruppe
   Ala-Glu-Leu-Ser-Pro-Ala-Ala-Lys-Thr-Gly-Lys-Arg-Lys-Arg. (aa. 121-134).
   Ala-Lys-Thr-Gly-Lys-Arg-Lys-Arg-Ser-Gln-Met-Leu-Phe-Gln. (aa. 127-140).

2. Verwendung der Peptide nach Anspruch 1, für die Bestimmung von Anti-IFN-γ-Antikörpern im Serum.

3. Verwendung nach Anspruch 2, worin die Bestimmung durch RIA, ELISA, Fluorimmunoassay-Verfahren durchgeführt wird.

4. Verwendung der Peptide nach Anspruch 1, für die Affinitätsreinigung von Anti-IFN-γ-Anitkörpern.

5. Verwendung von menschlichen Anti-IFN-γ-Antikörpern zur Herstellung eines Medikaments zur Behandlung von Bedingungen, welche von einer selektive Immununterdrückung von pathologischen Reaktionen, induziert durch IFN-γ, Nutzen ziehen können.

6. Verwendung nach Anspruch 5, worin die Bedingungen Diabetis vom Typ I, Multiple Sclerose, Lupus erythematosus, Adjuvant Arthritis, Schwartzmann-Reaktion, verzögerte Hypersensivität, Allotransplantatabstoßung sind.

7. Pharmazeutische Zusammensetzung, welche als aktiven Bestandteil menschliche Anti-IFN-γ-Antikörper enthält.

8. Pharmazeutische Zusammensetzung nach Anspruch 7 in Einheitsdosisform.

9. Verwendung von menschlichen Anti-IFN-γ-Antikörpern zur Reinigung von natürlichem oder rekombinantem IFN-γ.

10. Verwendung von menschlichen Anti-IFN-γ-Antikörpern für die Immunanalyse von IFN-γ.

**Revendications**

1. Peptides choisis dans le groupe
   Ala-Glu-Leu-Ser-Pro-Ala-Ala-Lys-Thr-Gly-Lys-Arg-Lys-Arg. (aa. 121-134)
   Ala-Lys-Thr-Gly-Lys-Arg-Lys-Arg-Ser-Gln-Met-Leu-Phe-Gln. (aa. 127-140)

2. Utilisation des peptides selon la revendication 1 pour la détermination d'anticorps anti-interféron-gamma dans le sérum.

3. Utilisation selon la revendication 2 dans laquelle la détermination est effectuée selon des méthodes radio-immunologiques, ELISA, fluoro-immunologiques.

4. Utilisation de peptides selon la revendication 1 pour la purification par affinité des anticorps anti-interféron-gamma.

5. Utilisation d'anticorps anti-interféron-gamma humains pour la préparation d'un médicament pour le traitement d'affections pouvant bénéficier d'une immunosuppression sélective des réponses pathologiques induites par interféron-gamma.

6. Utilisation selon la revendication 5 dans laquelle lesdites affections sont le diabète de type I, la sclérose en plaques, le lupus érythémateux, la polyarthrite, le phénomène de Schwartzmann-Sanarelli, l'hypersensibilité retardée, le rejet d'allogreffes.

7. Composition pharmaceutique contenant des anticorps anti-interféron-gamma humains en tant que constituant actif.

8. Composition pharmaceutique selon la revendication 7 sous forme de dose unitaire.

9. Utilisation d'anticorps anti-interféron-gamma pour la purification d'interféron-gamma naturelle ou recombinante.

10. Utilisation d'anticorps anti-interféron-gamma pour l'analyse immunologique d'interféron-gamma.

FIG. 1

FIG. 2

FIG. 3

17

FIG. 4

EP 0 485 471 B1

$MWx10^{-3}$     a   b   c   d   e   f   g

92.5–
69–
46–

30–

14.3–

FIG. 5

FIG. 6